# EUROPEAN PATENT SPECIFICATION

(11) **EP 2 617 714 B1**
(45) Date of publication and mention of the grant of the patent: **23.03.2016**
(21) Application number: 13159825.2
(22) Date of filing: 07.12.2006
(51) Int. Cl.: C07D 215/32, A61K 31/47, C07C 69/007, C07C 69/716, C07C 69/738, C07H 13/08, C07C 235/78, C07C 49/493, C07F 7/18, A61P 35/00

(54) **Chemical derivatives of jasmonate, pharmaceutical compositions and methods of use thereof**
Chemische Derivate aus Jasmonat, pharmazeutische Zusammensetzungen und Verwendungsverfahren dafür
Dérivés chimiques de jasmonate, compositions pharmaceutiques et leurs procédés d'utilisation

(30) Priority: 07.12.2005 US 742875 P; 13.02.2006 US 772567 P
(43) Date of publication of application: 24.07.2013
(62) Divisional of application: 11171983.7
(73) Proprietor: Ramot at Tel Aviv University, Ltd., 61392 Tel Aviv (IL)
(72) Inventor: Kashman, Yoel, 69548 Tel Aviv (IL); Flescher, Eliezer, 45272 Hod Hasharon (IL); Herzberg, Max, 76834 Sitrya (IL)
(74) Representative: Becker Kurig Straus

(56) References cited:
- WO-A-02/080890
- WO-A-2005/054172

## Description

### FIELD OF THE INVENTION

The present invention relates to the field of jasmonate derivative compounds, methods for their preparation, pharmaceutical compositions including such compounds, and methods of using these compounds and compositions, especially as chemotherapeutic agents for treatment of cancers especially in mammals, and particularly in humans.

### BACKGROUND OF THE INVENTION

Jasmonates are a family of plant stress hormones, derived from linolenic acid by the octadecanoid pathway, which are found in minute quantities in many edible plants. Stress hormones such as the jasmonate family have evolved in plants, and are released in such times of stress such as extreme UV radiation, osmotic shock, heat shock and pathogen attack, to initiate various cascades which end in appropriate responses. Examples of members of the jasmonate family are jasmonic acid, which is crucial to intracellular signaling in response to injury, and methyl jasmonate, which causes induction of a proteinase inhibitor that accumulates at low concentrations in response to wounding or pathogenic attacks. Use of jasmonates for the treatment of mammalian cancer has been disclosed in U.S. Patent No. 6,469,061. In U.S. Patent No. 6,469,061, it was shown that jasmonates were directly cytotoxic for various types of human cancer cells derived from breast, prostate, skin and blood cancers. While jasmonates elicited death in human leukemic Molt-4 cells, they did not damage normal lymphocytes.

In U.S. Patent No. 6,469,061, one jasmonate compound in particular, methyl jasmonate, was shown to be effective in preventing development of lymphomas in mice. See also Fingrut, O. and E. Flescher. 2002. "Plant stress hormones suppress the proliferation and induce apoptosis in human cancer cells", Leukemia 16: 608-616 (2002).

Subsequent data collected similarly showed that jasmonates do not damage healthy erythrocytes (see WO 02/080890).

PCT International Patent Publication WO 2005/054172 discloses novel halogenated jasmonate derivatives, pharmaceutical compositions comprising the derivatives, and their use for reducing cancer cell growth and for treating cancer.

Jasmonic acids conjugated via the carboxyl group to amino acids occur in nature (Plant Hormones, Davies PJ, ed., Kluwer Academic Publishers, London, 2004, pp. 618, 620). Several jasmonic acid-amino acid conjugates have been synthetically prepared. The amino acids include glycine, alanine, valine, leucine and isoleucine. (Jikumaru Y. et al. Biosci. Biotechnol. Biochem. 68, 1461-1466, 2004).

The pharmacological activity of jasmonate compounds makes them attractive candidates as therapeutic agents for the treatment of cancer. Only very few jasmonate derivatives have been reported in the art (see, for example, Ishii et al., Leukemia, 1-7 (2004); Seto et al. Biochem. Biosc. & Biotech. 63(2), (1999); Hossain et al. Biochem. Biosci. & Biotech. 68(9), 1842, (2004)). An unmet need exists to develop jasmonate derivative compounds that are potent chemotherapeutic drugs, with a high degree of specificity towards malignant cells.

### SUMMARY OF THE INVENTION

The present invention relates to novel jasmonate derivative compounds represented by the general structure of formula I, e.g., a compound of formula 9. Several of these compounds are significantly more potent than the compounds disclosed in U.S. 6,469,061 and WO 2005/054172. The novel derivatives exert selective cytotoxicity on cancerous cells, while spearing normal cells. As such, the compounds of the present invention are useful in inhibiting cancer cell proliferation and treating a variety of cancers.

In one embodiment, the jasmonate derivatives are represented by the structure of formula I. 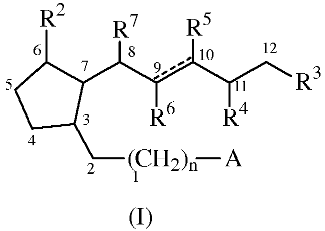 wherein in Formula I:
A is COR¹;
R¹ is a heteroaryloxy;
R² is selected from the group consisting of hydrogen, unsubstituted or substituted C₁-C₁₂ alkyl, unsubstituted or substituted C₃-C₈ cycloalkyl, unsubstituted or substituted aryl, unsubstituted or substituted heteroaryl, OR⁸, oxo and NR^{9a}R^{9b};
R³, R⁴, R⁵, R⁶ and R⁷ are each independently selected from the group consisting of hydrogen, unsubstituted or substituted C₁-C₁₂ alkyl, unsubstituted or substituted C₁-C₁₂ haloalkyl, unsubstituted or substituted C₃-C₈ cycloalkyl, unsubstituted or substituted aryl, unsubstituted or substituted heteroaryl, OR⁸ and NR^{9a}R^{9b},
or R⁵ and R⁶ together with the carbons to which they are attached form a C₃-C₈ cycloalkyl or a C₃-C₈ cycloalkyl substituted by halo;
or one of R⁵ and R⁶ represents an oxygen atom which is bonded to C₆, thereby forming an oxygen-containing 6 or 5 membered heterocyclic ring, respectively;
wherein the bond between C₉ and C₁₀ can be a single or double bond;
R⁸, R^{9a} and R^{9b} are each independently selected from the group consisting of hydrogen, unsubstituted or substituted C₁-C₁₂ alkyl, unsubstituted or substituted C₃-C₈ cycloalkyl, unsubstituted or substituted aryl, unsubstituted or substituted heteroaryl, glucosyl, or R^{9a} and R^{9b} can together with the nitrogen to which they are attached form an unsubstituted or substituted heterocyclic or heteroaromatic ring optionally containing one or more additional heteroatom selected from O, N and S; and
n is selected from 0, 1 and 2;
including salts, hydrates, solvates, polymorphs, optical isomers, geometrical isomers, enantiomers, diastereomers, and mixtures thereof.

In one embodiment, R¹ heteroaryloxy, preferably quinolinyloxy. The heteroaryloxy may be unsubstituted or substituted with one or more alkyl groups.

In one currently preferred embodiment, R² in formula (I) is oxo (=O). In one currently preferred embodiment, R⁵ and R⁶ together with the carbons to which they are attached (i.e., C₉ and C₁₀) form an unsubstituted C₃-C₈ cycloalkyl or a C₃-C₈ cycloalkyl substituted by one or more halogen atoms. In one specific embodiment, R⁵ and R⁶ together represent a C(Hal)₂ group wherein Hal is halogen, and together with C₉ and C₁₀ define a halo-substituted cyclopropyl group.

In another embodiment, each of R³, R⁴, R⁵, R⁶ and R⁷ is hydrogen. In another embodiment, the bond between C₉ and C₁₀ is a double bond, and each of R³, R⁴, R⁵, R⁶ and R⁷ is hydrogen. In yet another embodiment, the bond between C₉ and C₁₀ is a single bond, and each of R³, R⁴, R⁵, R⁶ and R⁷ is hydrogen. In yet another embodiment, R⁶ represents an oxygen atom which is bonded to C₆, thereby forming an oxygen-containing 5 membered heterocyclic ring.

An example of the compounds of formula I is a compound of formula 9:

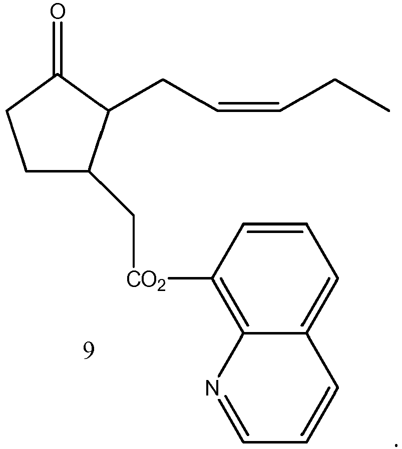

The present invention also contemplates pharmaceutical compositions that include a pharmaceutically acceptable carrier and, as an active ingredient, one or more of the compounds of the invention, represented by any of general formula I, e.g., compound 9, as described above.

The pharmaceutical compositions of the present invention can be provided in any form known in the art, for example in a form suitable for oral administration (e.g., a solution, a suspension, a syrup, an emulsion, a dispersion, a suspension, a tablet, a pill, a capsule, a pellet, granules and a powder), for parenteral administration (e.g., intravenous, intramuscular, intraarterial, transdermal, subcutaneous or intraperitoneal), for topical administration (e.g., an ointment, a gel, a cream), for administration by inhalation or for administration via suppository. Preferably, in the pharmaceutical composition of the present invention, the active ingredient is dissolved in any acceptable lipid carrier.

The jasmonate derivatives may be administered together with at least one other chemotherapeutic agent. The jasmonate derivative and the at least other chemotherapeutic agent can be administered simultaneously (in the same or in separate dosage forms), or they can be administered sequentially, in any order.

The present invention additionally provides a method for inhibiting cancer cell proliferation in vitro, comprising contacting the cancer cells with a compound of any of general formula I, e.g., formula 9as described herein. In some embodiments, the compound is administered in a pharmaceutical composition.

Furthermore, the present invention relates to a compound of formula I, e.g., formula 9according to the present invention for use in the treatment of cancer or a premalignant condition in a subject.

The compounds of the present invention are active against a wide range of cancers or premalignant conditions.

In one embodiment, the cancer is selected from the group consisting of: lymphoproliferative disorders, breast cancer, ovarian cancer, prostate cancer, cervical cancer, endometrial cancer, bone cancer, liver cancer, stomach cancer, colon cancer, pancreatic cancer, cancer of the thyroid, head and neck cancer, cancer of the central nervous system, cancer of the peripheral nervous system, skin cancer, kidney cancer, as well as metastases of all the above.

In another embodiment, the cancer is selected from the group consisting of: hepatocellular carcinoma, hepatoma, hepatoblastoma, rhabdomyosarcoma, esophageal carcinoma, thyroid carcinoma, ganglioblastoma, fibrosarcoma, myxosarcoma, liposarcoma, chondrosarcoma, osteogenic sarcoma, chordoma, angiosarcoma, endotheliosarcoma, Ewing's tumor, leimyosarcoma, rhabdotheliosarcoma, invasive ductal carcinoma, papillary adenocarcinoma, melanoma, squamous cell carcinoma, basal cell carcinoma, adenocarcinoma (well differentiated, moderately differentiated, poorly differentiated or undifferentiated), renal cell carcinoma, hypernephroma, hypernephroid adenocarcinoma, bile duct carcinoma, choriocarcinoma, seminoma, embryonal carcinoma, Wilms' tumor, testicular tumor, lung carcinoma including small cell, non-small and large cell lung carcinoma, bladder carcinoma, glioma, astrocyoma, medulloblastoma, craniopharyngioma, ependymoma, pinealoma, retinoblastoma, neuroblastoma, colon carcinoma, rectal carcinoma, hematopoietic malignancies including all types of leukemia and lymphoma including: acute myelogenous leukemia, acute myelocytic leukemia, acute lymphocytic leukemia, chronic myelogenous leukemia, chronic lymphocytic leukemia, mast cell leukemia, multiple myeloma, myeloid lymphoma, Hodgkin's lymphoma, non-Hodgkin's lymphoma.

In another embodiment, the cancer is selected from the group consisting of: prostate cancer, breast cancer, skin cancer, colon cancer, lung cancer, pancreatic cancer, lymphoma, myeloma, leukemia, head and neck cancer, kidney cancer, stomach cancer, ovarian cancer, bone cancer, liver cancer or thyroid cancer.

In another embodiment, the cancer is selected from the group consisting of: leukemia, including lymphoblastic leukemia, lung carcinoma, melanoma, kidney cancer, stomach cancer and colon cancer.

In another embodiment, the cancer is selected from the group consisting of: squamous cell carcinoma, basal cell carcinoma, skin cancer, head and neck cancer, esophageal carcinoma, rectal carcinoma, cervical cancer, as well as metastases of all of the above.

The jasmonate derivatives of the present invention are significantly more potent than the compounds disclosed in U.S. 6,469,061 and WO 2005/054172. They display an unexpected cytotoxic effect with a high degree of specificity towards malignant cells.

### BRIEF DESCRIPTION OF THE DRAWINGS

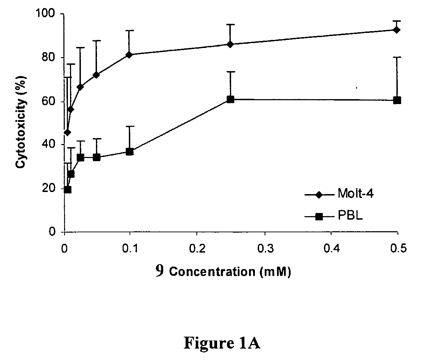

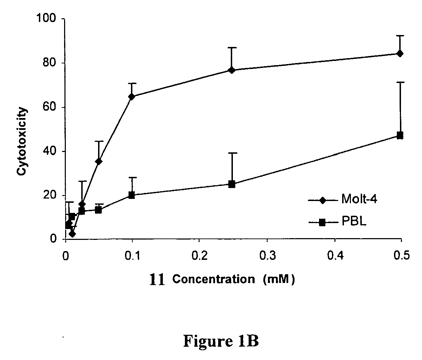

**FIGURE 1****:** Shows the cytotoxic activity of increasing concentrations (0.01-0.5 mM) of compounds 9 (Figure 1A) and 11 (Figure 1B) in lymphoblastic leukemia cells (Molt-4) and peripheral blood lymphocytes (PBL). Cytotoxicity (%) is plotted against the compound concentration.

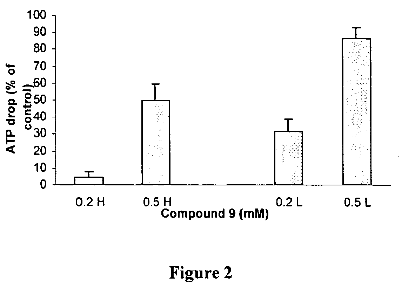

**FIGURE 2****:** Shows the ability of compound 9 to induce a decrease in the ATP levels of CT-26 colon carcinoma cells. Drop in ATP level in the cells after 3 hours incubation in different media is plotted; High (28mM) or low (2mM) glucose concentration with either low (0.2mM) or high (0.5mM) concentration of compound 9 is plotted.

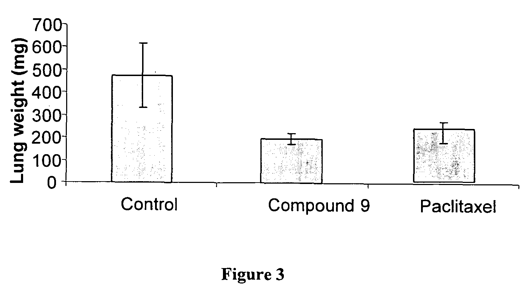

**FIGURE 3****:** Shows the ability of compound 9 to decrease experimental metastasis of B16 melanoma cells to the lungs in vivo. Lung weight of mice is plotted for a control group of untreated mice, a second group of mice treated with compound 9 and a third group of mice that were treated with Paclitaxel.

### DETAILED DESCRIPTION OF THE PRESENT INVENTION

The present invention relates to novel jasmonate derivative compounds represented by the general structure of formula I. An example of a compound of general formula I is a compound of formula 9. Some of these compounds are significantly more potent than the compounds disclosed in the art, and exert selective cytotoxicity on cancerous cells, e.g. lymphocytes, carcinoma cells and breast cancer cells, while having very low effect on normal cells. As such, the compounds of the present invention are useful in inhibiting cancer cell proliferation and treating a variety of cancers and premalignant conditions.

Also described are jasmonate derivatives represented by the structures 1-11 and their uses for inhibiting cancer cell proliferation, and treatment of cancer and premalignant conditions. In one embodiment, the compounds of the present invention are jasmonate derivatives represented by the general structure of formula I: 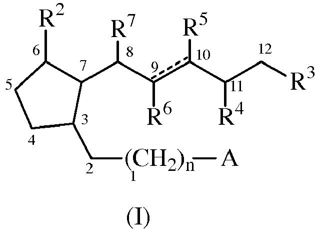 wherein in Formula I:
A is COR¹;
R¹ is a heteroaryloxy;
R² is selected from the group consisting of hydrogen, unsubstituted or substituted C₁-C₁₂ alkyl, unsubstituted or substituted C₃-C₈ cycloalkyl, unsubstituted or substituted aryl, unsubstituted or substituted heteroaryl, OR⁸, oxo and NR^{9a}R^{9b};
R³, R⁴, R⁵, R⁶ and R⁷ are each independently selected from the group consisting of hydrogen, unsubstituted or substituted C₁-C₁₂ alkyl, unsubstituted or substituted C₁-C₁₂ haloalkyl, unsubstituted or substituted C₃-C₈ cycloalkyl, unsubstituted or substituted aryl, unsubstituted or substituted heteroaryl, OR⁸ and NR^{9a}R^{9b},
or R⁵ and R⁶ together with the carbons to which they are attached form a C₃-C₈ cycloalkyl or a C₃-C₈ cycloalkyl substituted by halo;
or one of R⁵ and R⁶ represents an oxygen atom which is bonded to C₆, thereby forming an oxygen-containing 6 or 5 membered heterocyclic ring, respectively;
wherein the bond between C₉ and C₁₀ can be a single or double bond;
R⁸, R^{9a} and R^{9b} are each independently selected from the group consisting of hydrogen, unsubstituted or substituted C₁-C₁₂ alkyl, unsubstituted or substituted C₃-C₈ cycloalkyl, unsubstituted or substituted aryl, unsubstituted or substituted heteroaryl, glucosyl, or R^{9a} and R^{9b} can together with the nitrogen to which they are attached form an unsubstituted or substituted heterocyclic or heteroaromatic ring optionally containing one or more additional heteroatom selected from O, N and S; and
n is selected from 0, 1 and 2;

In one embodiment, R¹ heteroaryloxy, i.e. a heteroaryl moiety as described herein, linked to an oxygen. A currently preferred heteroaryloxy group is a quinolinyloxy group. The heteroaryloxy may be unsubstituted or substituted with one or more alkyl groups.

In one currently preferred embodiment, R² in formula (I) is oxo (=O In one currently preferred embodiment, R⁵ and R⁶ together with the carbons to which they are attached (i.e., C₉ and C₁₀) form an unsubstituted C₃-C₈ cycloalkyl or a C₃-C₈ cycloalkyl substituted by one or more halogen atoms. In one specific embodiment, R⁵ and R⁶ together represent a C(Hal)₂ group wherein Hal is halogen, and together with C₉ and C₁₀ define a halo-substituted cyclopropyl group.

Also described are compounds wherein R⁵ and R⁶ together with the carbons to which they are attached (i.e., C₉ and C₁₀) form an unsubstituted or substituted C₃-C₈ cycloalkyl. Preferred substituents for the C₃-C₈ cycloalkyl is C₁-C₁₂ alkyl or halogen. For example, for purposes of illustration and not for limitation, R⁵ and R⁶ can together define a (CRR')ₐ group wherein each of R and R' is independently a hydrogen, C₁-C₁₂ alkyl or halogen and "a" is an integer of 1-6, thereby forming a 3-8 cyclic ring together with C₉ and C₁₀. In one specific embodiment, R⁵ and R⁶ together represent a C(Hal)₂ group wherein Hal is halogen, and together with C₉ and C₁₀ define a halo-substituted cyclopropyl group. Also, as apparent to a person of skill in the art, any other substituents, together with the carbons to which they are attached, can similarly form a 3-8 membered cyclic structures. For example, any of the groups R³ and R⁴; R³ and R⁵; R³ and R⁶; R³ and R⁷; R⁴ and R⁵; R⁴ and R⁶; R⁴ and R⁷; R⁵ and R⁷ can together with the carbons to which they are attached form a cyclic structure in a similar manner as described above.

In one currently preferred embodiment, the bond between C₉ and C₁₀ is a double bond. In another currently preferred embodiment, the bond between C₉ and C₁₀ is a single bond. In another embodiment, each of R³, R⁴, R⁵, R⁶ and R⁷ represents hydrogen. In yet another embodiment, the bond between C₉ and C₁₀ is a single bond, and each of R³, R⁴, R⁵, R⁶ and R⁷ is hydrogen. In yet another embodiment, the bond between C₉ and C₁₀ is a double bond, and each of R³, R⁴, R⁵, R⁶ and R⁷ is hydrogen.

In yet another embodiment, R⁶ represents an oxygen atom which is bonded to C₆, thereby forming an oxygen-containing 5 membered heterocyclic ring. Also described are compounds wherein R⁵ represents an oxygen atom which is bonded to C₆, thereby forming an oxygen-containing 6 membered heterocyclic ring.

A specific example of the compounds of formula I includes a compound of formula 9. Also described are jasmonate derivatives are specific derivatives represented by the structures 1-8 and 10-11, which do not form part of the present invention. The structures of these compounds are shown hereinbelow:

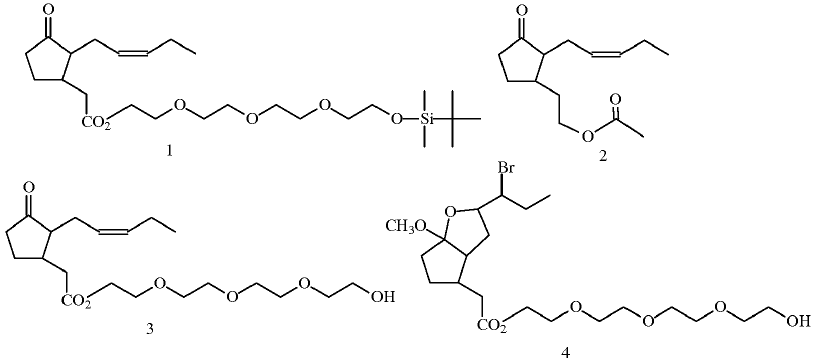

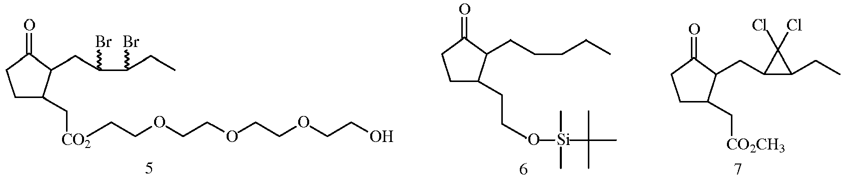

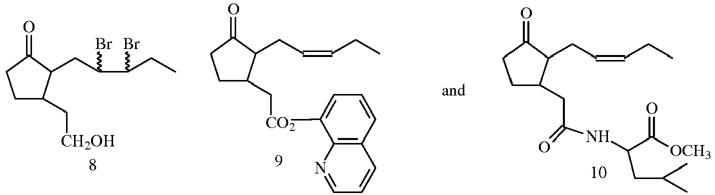

Also described is a jasmonate derivative represented by the structure of formula 11. 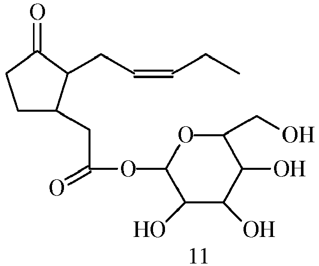 including salts, hydrates, solvates, polymorphs, optical isomers, geometrical isomers, enantiomers, diastereomers, and mixtures thereof. As demonstrated herein, said compound has unexpectedly been found to be a highly potent and selective cytotoxic agent, exhibiting selective cytotoxicity towards cancer cells, while having little effect on normal cells. As such, compound 11 possesses surprisingly superior properties as compared with the jasmonate glucosyl derivatives disclosed in U.S. 6,469,061.

### Chemical Definitions

The term "C₁ to C₁₂ alkyl" used herein alone or as part of another group denotes linear and branched, Preferred are alkyl groups containing from 1 to 4 carbon atoms (C₁ to C₄ alkyls). Examples of saturated alkyl groups include but are not limited to methyl, ethyl, n-propyl, isopropyl, n-butyl, iso-butyl, sec-butyl, tert-butyl, amyl, tert-amyl, hexyl, heptyl, octyl, nonyl, decyl, undecyl, dodecyl and the like.

The C₁ to C₁₂ alkyl group can be unsubstituted, or substituted with one or more substituents selected from the group consisting of halogen, hydroxy, alkoxy, aryloxy, alkylaryloxy, heteroaryloxy, oxo, cycloalkyl, phenyl, heteroaryl, heterocyclyl, naphthyl, amino, alkylamino, arylamino, heteroarylamino, dialkylamino, diarylamino, alkylarylamino, alkylheteroarylamino, arylheteroarylamino, acyl, acyloxy, nitro, carboxy, carbamoyl, carboxamide, cyano, sulfonyl, sulfonylamino, sulfinyl, sulfinylamino, thiol, C₁ to C₁₀ alkylthio arylthio, or C₁ to C₁₀ alkylsulfonyl groups. Any substituent can be unsubstituted or further substituted with any one of these aforementioned substituents.

The term "C₃ to C₈ cycloalkyl" used herein alone or as part of another group denotes any monocyclic or polycyclic group. Nonlimiting examples of cycloalkyl groups are cyclopropyl, cyclobutyl, cyclopentyl, cyclohexyl or cycloheptyl. The cycloalkyl group can be unsubstituted or substituted with any one or more of the substituents defined above for alkyl.

The term "aryl" used herein alone or as part of another group denotes an aromatic ring system containing from 6-14 ring carbon atoms. The aryl ring can be a monocyclic, bicyclic, tricyclic and the like. Non-limiting examples of aryl groups are phenyl, naphthyl including 1-naphthyl and 2-naphthyl, and the like. The aryl group can be unsubtituted or substituted through available carbon atoms with one or more groups defined hereinabove for alkyl.

The term "heteroaryl" used herein alone or as part of another group denotes a heteroaromatic system containing at least one heteroatom ring atom selected from nitrogen, sulfur and oxygen. The heteroaryl contains 5 or more ring atoms. The heteroaryl group can be monocyclic, bicyclic, tricyclic and the like. Also included in this expression are the benzoheterocyclic rings. If nitrogen is a ring atom, the present invention also contemplates the N-oxides of the nitrogen containing heteroaryls. Nonlimiting examples of heteroaryls include thienyl, benzothienyl, 1-naphthothienyl, thianthrenyl, furyl, benzofuryl, pyrrolyl, imidazolyl, pyrazolyl, pyridyl, pyrazinyl, pyrimidinyl, pylidazinyl, indolyl, isoindolyl, indazolyl, purinyl, quinolyl (e.g. 1-quinolinyl, 2-quinolinyl, 3-quinolinyl, 4-quinolinyl, 5-quinolinyl, 6-quinolinyl, 7-quinolinyl and 8-quinolinyl), isoquinolinyl (e.g., 1-isoquinolinyl, 2-isoquinolinyl, 3-isoquinolinyl, 4-isoquinolinyl, 5-isoquinolinyl, 6-isoquinolinyl, 7-isoquinolinyl and 8-isoquinolinyl); naphthyridinyl (e.g., 1-naphthyridinyl, 2-naphthyridinyl), quinoxalinyl, quinazolinyl, cinnolinyl, pteridinyl, carbolinyl, thiazolyl, oxazolyl, isothiazolyl, isoxazolyl and the like. The heteroaryl group can optionally be substituted through available atoms with one or more groups defined hereinabove for alkyl. The heteroaryl group can be unsubtituted or substituted through available atoms with one or more groups defined hereinabove for alkyl.

The term "heterocyclic ring" or "heterocyclyl" used herein alone or as part of another group denotes a five-membered to eight-membered rings that have 1 to 4 heteroatoms, such as oxygen, sulfur and/or nitrogen, in particular nitrogen, either alone or in conjunction with sulfur or oxygen ring atoms. These five-membered to eight-membered rings can be saturated, fully unsaturated or partially unsaturated, with fully saturated rings being preferred. Preferred heterocyclic rings include piperidinyl, piperidinyl, pyrrolidinyl pyrrolinyl, pyrazolinyl, pyrazolidinyl, piperidinyl, morpholinyl, thiomorpholinyl, pyranyl, thiopyranyl, piperazinyl, indolinyl, dihydrofuranyl, tetrahydrofuranyl, dihydrothiophenyl, tetrahydrothiophenyl, dihydropyranyl, tetrahydropyranyl, and the like. The heterocyclyl group can be unsubtituted or substituted through available atoms with one or more groups defined hereinabove for alkyl.

The term "halogen" or "halo" as used herein alone or as part of another group refers to chlorine, bromine, fluorine, and iodine.

The term "amino" as used herein alone or as part of another group refers to an NH₂ group. The terms "alkyl amino, dialkylamino, arylamino, diaryl amino, heteroarylamino, diheteroarylamino" and variants thereof as used herein refer to amino substituted with one or two substituents, which may be the same or different, such as alkyl, aryl, arylalkyl, heteroaryl, heteroarylalkyl, cycloheteroalkyl, cycloheteroalkylalkyl, cycloalkyl, cycloalkylalkyl, haloalkyl, hydroxyalkyl, alkoxyalkyl, thioalkyl and the like. These substituents can be further substituted with any one or more of the substituents defined above for alkyl. In addition, the amino substituents (e.g., NR^{9a}R^{9b}) can together with the nitrogen atom to which they are attached form a heterocyclic ring which can be any one of the heterocyclic rings defined above.

The term "hydroxy" refers to an OH group. The terms "alkoxy", "aryloxy" "arylalkyloxy" or "heteroaryloxy" as used herein alone or as part of another group includes any of the above alkyl, aryl or heteroaryl groups linked to an oxygen atom. Nonlimiting examples of an alkoxy group is methoxy, ethoxy, n-propoxy, isopropoxy, n-butoxy, t-butoxy and like groups. An example of an aryloxy group is phenyloxy (phenoxy). The alkoxy, aryloxy, arylalkyloxy or heteroaryloxy groups can be unsubstituted or substituted with any one or more of the substituents defined above for alkyl.

The term "carboxy" as used herein alone or as part of another group refers to a COO group, and further encompasses carboxylate salts thereof of the formula COOM wherein M is a metal ion. The term "metal ion" refers to alkali metal ions such as sodium, potassium or lithium and alkaline earth metal ions such as magnesium and calcium, as well as zinc and aluminum.

The term "acyl" encompasses groups such as formyl, acetyl, propionyl, butyryl, pentanoyl, pivaloyl, hexanoyl, heptanoyl, octanoyl, nonanoyl, decanoyl, undecanoyl, dodecanoyl, benzoyl and the like. Preferred acyl groups are acetyl and benzoyl.

The term "thio" as used herein alone or as part of another group refers to an SH group. The terms "alkylthio", "arylthio" or "arylalkylthio" as used herein alone or as part of another group refer to any of the above alkyl, arylalkyl or aryl groups linked to a sulfur atom.

The term "sulfonyl" as used herein alone or as part of another group refers to-S(O)₂-. The term "sulfonylamino" as used herein alone or as part of another group refers to -S(O)₂-NH. The term "sulfinyl" refers to -S(O)-. The term "sulfinylamino" as used herein alone or as part of another group refers to -S(O)-NH. The term "oxo" as used herein alone or as part of another group refers to -O-. The term "cyano" as used herein alone or as part of another group refers to a CN group. The term "nitro" as used herein alone or as part of another group refers to an NO₂ group.

All stereoisomers of the compounds of the instant invention are contemplated, either in admixture or in pure or substantially pure form. The compounds of the present invention can have asymmetric centers at any of the atoms. Consequently, the compounds can exist in enantiomeric or diastereomeric forms or in mixtures thereof. The present invention contemplates the use of any racemates (i.e. mixtures containing equal amounts of each enantiomers), enantiomerically enriched mixtures (i.e., mixtures enriched for one enantiomer), pure enantiomers or diastereomers, or any mixtures thereof. The chiral centers can be designated as R or S or R,S or d,D, 1,L or d,l, D,L. Compounds comprising amino acid residues include residues of D-amino acids, L-amino acids, or racemic derivatives of amino acids. Compounds comprising sugar residues include residues of D-sugars, L-sugars, or racemic derivatives of sugars. In addition, several of the compounds of the invention contain one or more double bonds. The present invention intends to encompass all structural and geometrical isomers including cis, trans, E and Z isomers.

One or more of the compounds of the invention, may be present as a salt. The term "salt" encompasses both basic and acid addition salts, including but not limited to carboxylate salts or salts with amine nitrogens, and include salts formed with the organic and inorganic anions and cations discussed below. Furthermore, the term includes salts that form by standard acid-base reactions with basic groups (such as amino groups) and organic or inorganic acids. Such acids include hydrochloric, hydrofluoric, trifluoroacetic, sulfuric, phosphoric, acetic, succinic, citric, lactic, maleic, fumaric, palmitic, cholic, pamoic, mucic, D-glutamic, D-camphoric, glutaric, phthalic, tartaric, lauric, stearic, salicyclic, methanesulfonic, benzenesulfonic, sorbic, picric, benzoic, cinnamic, and like acids.

The term "organic or inorganic cation" refers to counter-ions for the carboxylate anion of a carboxylate salt. The counter-ions are chosen from the alkali and alkaline earth metals, (such as lithium, sodium, potassium, barium, aluminum and calcium); ammonium and mono-, di- and tri-alkyl amines such as trimethylamine, cyclohexylamine; and the organic cations, such as dibenzylammonium, benzylammonium, 2-hydroxyethylammonium, bis(2-hydroxyethyl)ammonium, phenylethylbenzylammonium, dibenzylethylenediammonium, and like cations. See, for example, "Pharmaceutical Salts," Berge et al., J. Pharm. Sci., 66:1-19 (1977). Other cations encompassed by the above term include the protonated form of procaine, quinine and N-methylglucosamine, and the protonated forms of basic amino acids such as glycine, ornithine, histidine, phenylglycine, lysine and arginine. Furthermore, any zwitterionic form of the instant compounds formed by a carboxylic acid and an amino group are also contemplated.

The present invention also includes solvates of the compounds of the present invention and salts thereof. "Solvate" means a physical association of a compound of the invention with one or more solvent molecules. This physical association involves varying degrees of ionic and covalent bonding, including hydrogen bonding. In certain instances the solvate will be capable of isolation. "Solvate" encompasses both solution-phase and isolatable solvates. Non-limiting examples of suitable solvates include ethanolates, methanolates and the like. "Hydrate" is a solvate wherein the solvent molecule is water.

The present invention also includes polymorphs of the compounds of the present invention and salts thereof. The term "polymorph" refers to a particular crystalline state of a substance, which can be characterized by particular physical properties such as X-ray diffraction, IR spectra, melting point, and the like.

### Therapeutic Use

As described herein, the compounds of the present invention are potent cytotoxic agents that are capable of inhibiting cancer cell proliferation in a wide variety of cancer cells. Described are powerful methods to the chemoprevention and treatment of cancer that have not been previously described.

Thus, in one aspect the present invention additionally provides a method for inhibiting cancer cell proliferation in vitro, comprising contacting the cancer cells with a compound of the present invention, as described herein. The compound is one or more of the compounds represented by formula I, e.g., formula 9 as described herein. In some embodiments, the compound is administered in a pharmaceutical composition.

Further described are methods for the treatment of cancer in a subject, by administering to the subject a therapeutically effective amount of the compound of the invention, as described herein. Preferably, the compound is one or more of the compounds represented by formula I, e.g., formula 9, as described herein. In some embodiments, the compound is administered in a pharmaceutical composition. In one embodiment, the subject is a mammal, preferably a human. The present invention also contemplates using the compounds of the present invention for non-human mammals, e.g., in veterinary medicine.

Furthermore, the present invention relates to the use of a compound of formula I, e.g., formula 9 according to the present invention in the preparation of a medicament useful for the treatment of cancer or a premalignant condition.

Furthermore, the present invention relates to a compound of formula I, e.g., formula 9 according to the present invention for use in the treatment of cancer or a premalignant condition in a subject.

The compounds of the present invention are active against a wide range of cancers or premalignant conditions. It is to be understood that whenever the terms "treating or inhibiting a malignant cell proliferative disease or disorder", "treating or inhibiting a non-solid cancer", "treating or inhibiting a tumor" are used herein in the description and in the claims, they are intended to encompass tumor formation, primary tumors, tumor progression or tumor metastasis.

The term "inhibition of proliferation" in relation to cancer cells, in the context of the present invention refers to a decrease in at least one of the following: number of cells (due to cell death which may be necrotic, apoptotic or any other type of cell death or combinations thereof) as compared to control; decrease in growth rates of cells, i.e. the total number of cells may increase but at a lower level or at a lower rate than the increase in control; decrease in the invasiveness of cells (as determined for example by soft agar assay) as compared to control even if their total number has not changed; progression from a less differentiated cell type to a more differentiated cell type; a deceleration in the neoplastic transformation; or alternatively the slowing of the progression of the cancer cells from one stage to the next.

The term "treatment of cancer" in the context of the present invention includes at least one of the following: a decrease in the rate of growth of the cancer (i.e. the cancer still grows but at a slower rate); cessation of growth of the cancerous growth, i.e., stasis of the tumor growth, and, in preferred cases, the tumor diminishes or is reduced in size. The term also includes reduction in the number of metastasis, reduction in the number of new metastasis formed, slowing of the progression of cancer from one stage to the other and a decrease in the angiogenesis induced by the cancer. In most preferred cases, the tumor is totally eliminated. Additionally included in this term is lengthening of the survival period of the subject undergoing treatment, lengthening the time of diseases progression, tumor regression, and the like. This term also encompasses prevention for prophylactic situations or for those individuals who are susceptible to contracting a tumor. The administration of the compounds of the present invention will reduce the likelihood of the individual contracting the disease. In preferred situations, the individual to whom the compound is administered does not contract the disease.

As used herein, the term "administering" refers to bringing in contact with a compound of the present invention. Administration can be accomplished to cells or tissue cultures, or to living organisms, for example humans. In one embodiment, the present invention encompasses administering the compounds of the present invention to a human subject.

A "therapeutic" treatment is a treatment administered to a subject who exhibits signs of pathology for the purpose of diminishing or eliminating those signs. A "therapeutically effective amount" of a compound of the invention is that amount of compound which is sufficient to provide a beneficial effect to the subject to which the compound is administered.

The term "cancer" in the context of the present invention includes all types of neoplasm whether in the form of solid or non-solid tumors, and includes both malignant and premalignant conditions as well as their metastasis.

Cancers may be classified in two ways: by the type of tissue in which the cancer originates (histological type) and by primary site, or the location in the body where the cancer first developed. The international standard for the classification and nomenclature of histologies is the International Classification of Diseases for Oncology, Third Edition.

From a histological standpoint there are hundreds of different cancers, which are grouped into five major categories: carcinoma, sarcoma, myeloma, leukemia, and lymphoma. In addition, there are also some cancers of mixed types.

Carcinoma refers to a malignant neoplasm of epithelial origin or cancer of the internal or external lining of the body. Carcinomas, malignancies of epithelial tissue, account for 80 to 90 percent of all cancer cases. Epithelial tissue is found throughout the body. It is present in the skin, as well as the covering and lining of organs and internal passageways, such as the gastrointestinal tract.

Carcinomas are divided into two major subtypes: adenocarcinoma, which develops in an organ or gland, and squamous cell carcinoma, which originates in the squamous epithelium. Most carcinomas affect organs or glands capable of secretion, such as the breasts, which produce milk, or the lungs, which secrete mucus, or colon or prostate or bladder.

Adenocarcinomas generally occur in mucus membranes and are first seen as a thickened plaque-like white mucosa. They often spread easily through the soft tissue where they occur. Squamous cell carcinomas occur in many areas of the body.

Sarcoma refers to cancer that originates in supportive and connective tissues such as bones, tendons, cartilage, muscle, and fat. Generally occurring in young adults, the most common sarcoma often develops as a painful mass on the bone. Sarcoma tumors usually resemble the tissue in which they grow.

Examples of sarcomas are: Osteosarcoma or osteogenic sarcoma (bone); Chondrosarcoma (cartilage); Leiomyosarcoma (smooth muscle); Rhabdomyosarcoma (skeletal muscle); Mesothelial sarcoma or mesothelioma (membranous lining of body cavities); Fibrosarcoma (fibrous tissue); Angiosarcoma or hemangioendothelioma (blood vessels); Liposarcoma (adipose tissue); Glioma or astrocytoma (neurogenic connective tissue found in the brain); Myxosarcoma (primitive embryonic connective tissue); Mesenchymous or mixed mesodermal tumor (mixed connective tissue types);

Myeloma is cancer that originates in the plasma cells of bone marrow. The plasma cells produce some of the proteins found in blood.

Leukemias ("non-solid tumors" or "blood cancers") are cancers of the bone marrow (the site of blood cell production). The disease is often associated with the overproduction of immature white blood cells. Leukemia also affects red blood cells and can cause poor blood clotting and fatigue due to anemia. Examples of leukemia include: Myelogenous or granulocytic leukemia (malignancy of the myeloid and granulocytic white blood cell series); Lymphatic, lymphocytic, or lymphoblastic leukemia (malignancy of the lymphoid and lymphocytic blood cell series); Polycythemia vera or erythremia (malignancy of various blood cell products, but with red cells predominating)

Lymphomas develop in the glands or nodes of the lymphatic system, a network of vessels, nodes, and organs (specifically the spleen, tonsils, and thymus) that purify bodily fluids and produce infection-fighting white blood cells, or lymphocytes. Unlike the leukemias, which are sometimes called "non-solid tumors," lymphomas are "solid cancers." Lymphomas may also occur in specific organs such as the stomach, breast or brain. These lymphomas are referred to as extranodal lymphomas. The lymphomas are subclassified into two categories: Hodgkin lymphoma and Non-Hodgkin lymphoma. The presence of Reed-Sternberg cells in Hodgkin lymphoma diagnostically distinguishes Hodgkin lymphoma from Non-Hodgkin lymphoma.

Mixed Type cancers contain several types of cells. The type components may be within one category or from different categories. Some examples are: adenosquamous carcinoma; mixed mesodermal tumor; carcinosarcoma; teratocarcinoma

As used herein, the term "cancer" includes the above categories of carcinoma, sarcoma, myeloma, leukemia, lymphoma and mixed type tumors. In particular, the term cancer includes: lymphoproliferative disorders, breast cancer, ovarian cancer, prostate cancer, cervical cancer, endometrial cancer, bone cancer, liver cancer, stomach cancer, colon cancer, pancreatic cancer, cancer of the thyroid, head and neck cancer, cancer of the central nervous system, cancer of the peripheral nervous system, skin cancer, kidney cancer, as well as metastases of all the above. More particularly, as used herein the term may refer to: hepatocellular carcinoma, hematoma, hepatoblastoma, rhabdomyosarcoma, esophageal carcinoma, thyroid carcinoma, ganglioblastoma, fibrosarcoma, myxosarcoma, liposarcoma, chondrosarcoma, osteogenic sarcoma, chordoma, angiosarcoma, endotheliosarcoma, Ewing's tumor, leimyosarcoma, rhabdotheliosarcoma, invasive ductal carcinoma, papillary adenocarcinoma, melanoma, squamous cell carcinoma, basal cell carcinoma, adenocarcinoma (well differentiated, moderately differentiated, poorly differentiated or undifferentiated), renal cell carcinoma, hypernephroma, hypernephroid adenocarcinoma, bile duct carcinoma, choriocarcinoma, seminoma, embryonal carcinoma, Wilms' tumor, testicular tumor, lung carcinoma including small cell, non-small and large cell lung carcinoma, bladder carcinoma, glioma, astrocyoma, medulloblastoma, craniopharyngioma, ependymoma, pinealoma, retinoblastoma, neuroblastoma, colon carcinoma, rectal carcinoma, hematopoietic malignancies including all types of leukemia and lymphoma including: acute myelogenous leukemia, acute myelocytic leukemia, acute lymphocytic leukemia, chronic myelogenous leukemia, chronic lymphocytic leukemia, mast cell leukemia, multiple myeloma, myeloid lymphoma, Hodgkin's lymphoma, non-Hodgkin's lymphoma.

In one embodiment, the cancer is selected from the group consisting of: lymphoproliferative disorders, breast cancer, ovarian cancer, prostate cancer, cervical cancer, endometrial cancer, bone cancer, liver cancer, stomach cancer, colon cancer, pancreatic cancer, cancer of the thyroid, head and neck cancer, cancer of the central nervous system, cancer of the peripheral nervous system, skin cancer, kidney cancer, as well as metastases of all the above.

In another embodiment, the cancer is selected from the group consisting of: hepatocellular carcinoma, hepatoma, hepatoblastoma, rhabdomyosarcoma, esophageal carcinoma, thyroid carcinoma, ganglioblastoma, fibrosarcoma, myxosarcoma, liposarcoma, chondrosarcoma, osteogenic sarcoma, chordoma, angiosarcoma, endotheliosarcoma, Ewing's tumor, leimyosarcoma, rhabdotheliosarcoma, invasive ductal carcinoma, papillary adenocarcinoma, melanoma, squamous cell carcinoma, basal cell carcinoma, adenocarcinoma (well differentiated, moderately differentiated, poorly differentiated or undifferentiated), renal cell carcinoma, hypernephroma, hypernephroid adenocarcinoma, bile duct carcinoma, choriocarcinoma, seminoma, embryonal carcinoma, Wilms' tumor, testicular tumor, lung carcinoma including small cell, non-small and large cell lung carcinoma, bladder carcinoma, glioma, astrocyoma, medulloblastoma, craniopharyngioma, ependymoma, pinealoma, retinoblastoma, neuroblastoma, colon carcinoma, rectal carcinoma, hematopoietic malignancies including all types of leukemia and lymphoma including: acute myelogenous leukemia, acute myelocytic leukemia, acute lymphocytic leukemia, chronic myelogenous leukemia, chronic lymphocytic leukemia, mast cell leukemia, multiple myeloma, myeloid lymphoma, Hodgkin's lymphoma, non-Hodgkin's lymphoma.

In another embodiment, the cancer is selected from the group consisting of: prostate cancer, breast cancer, skin cancer, colon cancer, lung cancer, pancreatic cancer, lymphoma, myeloma, leukemia, head and neck cancer, kidney cancer, stomach cancer, ovarian cancer, bone cancer, liver cancer or thyroid cancer.

In another embodiment, the cancer is selected from the group consisting of: leukemia, including lymphoblastic leukemia, lung carcinoma, melanoma, kidney cancer, stomach cancer and colon cancer.

In another embodiment, the cancer is selected from the group consisting of: squamous cell carcinoma, basal cell carcinoma, skin cancer, head and neck cancer, esophageal carcinoma, rectal carcinoma, cervical cancer, as well as metastases of all of the above.

The cancer may also be selected from the group consisting of prostate cancer, breast cancer, skin cancer, colon cancer, lung cancer, pancreatic cancer, lymphoma, myeloma, leukemia, head and neck cancer, kidney cancer, stomach cancer, ovarian cancer, bone cancer, liver cancer or thyroid cancer. Even more preferably, the cancer is selected from leukemia, including lymphoblastic leukemia, lung carcinoma, melanoma, kidney cancer, stomach cancer and colon cancer.

Regarding the use of preparing a medicament, the medicament may additionally comprise at least one active chemotherapeutic agent other than the compounds of the invention. In certain embodiments, the compounds of the invention may be administered alongside with at least one traditional chemotherapeutic drug that is effective at treating the particular cancer. The administration can be concurrent (either combined in one dosage form or in separate dosage forms) or sequential. If provided sequentially, the jasmonate derivative can be administered before or after treatment with the additional chemotherapeutic agent(s). The combination of a compound of the invention and the traditional drug may allow administration of a lower dosage of the traditional drug, and thus the side effects experienced by the subject may be significantly lower, while a sufficient chemotherapeutic effect is nevertheless achieved.

### Pharmaceutical Compositions

Although the heterocyclic jasmonate derivatives of the present invention can be administered alone, it is contemplated that these compounds will be administered in a pharmaceutical composition containing the jasmonate derivative together with a pharmaceutically acceptable carrier or excipient.

Preferably, in the pharmaceutical composition the active ingredient is dissolved in any acceptable lipid carrier (e.g., fatty acids, oils to form, for example, a micelle or a liposome). Further, in accordance with a preferred embodiment of the present invention, the composition additionally comprises at least one other chemotherapeutic agent

The pharmaceutical compositions of the present invention can be formulated for administration by a variety of routes including oral, rectal, transdermal, parenteral (subcutaneous, intraperitoneal, intravenous, intraarterial, transdermal and intramuscular), topical, intranasal, or via a suppository. Such compositions are prepared in a manner well known in the pharmaceutical art and comprise as an active ingredient at least one compound of the present invention as described hereinabove, and a pharmaceutically acceptable excipient or a carrier. The term "pharmaceutically acceptable" means approved by a regulatory agency of the Federal or a state government or listed in the U. S. Pharmacopeia or other generally recognized pharmacopeia for use in animals and, more particularly, in humans.

During the preparation of the pharmaceutical compositions according to the present invention the active ingredient is usually mixed with a carrier or excipient, which may be a solid, semi-solid, or liquid material. The compositions can be in the form of tablets, pills, capsules, pellets, granules, powders, lozenges, sachets, cachets, elixirs, suspensions, dispersions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

The carriers may be any of those conventionally used and are limited only by chemical-physical considerations, such as solubility and lack of reactivity with the compound of the invention, and by the route of administration. The choice of carrier will be determined by the particular method used to administer the pharmaceutical composition. Some examples of suitable carriers include lactose, glucose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water and methylcellulose. The formulations can additionally include lubricating agents such as talc, magnesium stearate, and mineral oil; wetting agents, surfactants, emulsifying and suspending agents; preserving agents such as methyl- and propylhydroxybenzoates; sweetening agents; flavoring agents, colorants, buffering agents (e.g., acetates, citrates or phosphates), disintegrating agents, moistening agents, antibacterial agents, antioxidants (e.g., ascorbic acid or sodium bisulfite), chelating agents (e.g., ethylenediaminetetraacetic acid), and agents for the adjustment of tonicity such as sodium chloride. Other pharmaceutical carriers can be sterile liquids, such as water and oils, including those of petroleum, animal, vegetable or synthetic origin, such as peanut oil, soybean oil, mineral oil, sesame oil and the like, polyethylene glycols, glycerine, propylene glycol or other synthetic solvents. Water is a preferred carrier when the pharmaceutical composition is administered intravenously. Saline solutions and aqueous dextrose and glycerol solutions can also be employed as liquid carriers, particularly for injectable solutions.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

Any method can be used to prepare the pharmaceutical compositions. Solid dosage forms can be prepared by wet granulation, dry granulation, direct compression and the like.

The solid dosage forms of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer, which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the compositions of the present invention may be incorporated, for administration orally or by injection, include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insulation include solutions and suspensions in pharmaceutically acceptable aqueous or organic solvents, or mixtures thereof, and powders. The liquid or solid compositions may contain suitable pharmaceutically acceptable excipients as described above. Preferably the compositions are administered by the oral or nasal respiratory route for local or systemic effect. Compositions in preferably pharmaceutically acceptable solvents may be nebulized by use of inert gases. Nebulized solutions may be breathed directly from the nebulizing device or the nebulizing device may be attached to a face masks tent, or intermittent positive pressure breathing machine. Solution, suspension, or powder compositions may be administered, preferably orally or nasally, from devices that deliver the formulation in an appropriate manner.

Another formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art.

In yet another embodiment, the composition is prepared for topical administration, e.g. as an ointment, a gel a drop or a cream. For topical administration to body surfaces using, for example, creams, gels, drops, ointments and the like, the compounds of the present invention can be prepared and applied in a physiologically acceptable diluent with or without a pharmaceutical carrier. The present invention may be used topically or transdermally to treat cancer, for example, melanoma. Adjuvants for topical or gel base forms may include, for example, sodium carboxymethylcellulose, polyacrylates, polyoxyethylene-polyoxypropylene-block polymers, polyethylene glycol and wood wax alcohols.

Alternative formulations include nasal sprays, liposomal formulations, slow-release formulations, controlled-release formulations and the like, as are known in the art.

The compositions are preferably formulated in a unit dosage form. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

In preparing a formulation, it may be necessary to mill the active ingredient to provide the appropriate particle size prior to combining with the other ingredients. If the active compound is substantially insoluble, it ordinarily is milled to a particle size of less than 200 mesh. If the active ingredient is substantially water soluble, the particle size is normally adjusted by milling to provide a substantially uniform distribution in the formulation, e.g. about 40 mesh.

It may be desirable to administer the pharmaceutical composition of the invention locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material. According to some preferred embodiments, administration can be by direct injection e.g., via a syringe, at the site of a tumor or neoplastic or pre-neoplastic tissue.

The compounds may also be administered by any convenient route, for example by infusion or bolus injection, by absorption through epithelial linings (e.g., oral mucosa, rectal and intestinal mucosa, etc.), and may be administered together with other therapeutically active agents. It is preferred that administration is localized, but it may be systemic. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir. Pulmonary administration can also be employed, e.g., by use of an inhaler or nebulizer, and formulation with an aerosolizing agent.

A compound of the present invention can be delivered in an immediate release or in a controlled release system. In one embodiment, an infusion pump may be used to administer a compound of the invention, such as one that is used for delivering chemotherapy to specific organs or tumors (see Buchwald et al., 1980, Surgery 88: 507; Saudek et al., 1989, N. Engl. J. Med. 321: 574). In a preferred form, a compound of the invention is administered in combination with a biodegradable, biocompatible polymeric implant, which releases the compound over a controlled period of time at a selected site. Examples of preferred polymeric materials include polyanhydrides, polyorthoesters, polyglycolic acid, polylactic acid, polyethylene vinyl acetate, copolymers and blends thereof (See, Medical applications of controlled release, Langer and Wise (eds.), 1974, CRC Pres., Boca Raton, Fla.). In yet another embodiment, a controlled release system can be placed in proximity of the therapeutic target, thus requiring only a fraction of the systemic dose.

Furthermore, at times, the pharmaceutical compositions may be formulated for parenteral administration (subcutaneous, intravenous, intraarterial, transdermal, intraperitoneal or intramuscular injection) and may include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that include suspending agents, solubilizers, thickening agents, stabilizers, and preservatives. Oils such as petroleum, animal, vegetable, or synthetic oils and soaps such as fatty alkali metal, ammonium, and tuethanolamine salts, and suitable detergents may also be used for parenteral administration. The above formulations may also be used for direct intra-tumoral injection. Further, in order to minimize or eliminate irritation at the site of injection, the compositions may contain one or more nonionic surfactants. Suitable surfactants include polyethylene sorbitan fatty acid esters, such as sorbitan monooleate and the high molecular weight adducts of ethylene oxide with a hydrophobic base, formed by the condensation of propylene oxide with propylene glycol.

The parenteral formulations can be presented in unit-dose or multi-dose sealed containers, such as ampoules and vials, and can be stored in a freeze-dried (lyophilized) condition requiring only the addition of the sterile liquid carrier, for example, water, for injections, immediately prior to use. Extemporaneous injection solutions and suspensions can be prepared from sterile powders, granules, and tablets of the kind previously described and known in the art.

Alternatively, the jasmonate derivatives of the present invention can be used in hemodialysis such as leukophoresis and other related methods, e.g., blood is drawn from the patient by a variety of methods such dialysis through a column/hollow fiber membrane, cartridge etc, is treated with the jasmonate derivatives Ex-vivo, and returned to the patient following treatment. Such treatment methods are well known and described in the art. See, e.g., Kolho et al. (J. Med. Virol. 1993, 40(4): 318-21); Ting et al. (Transplantation, 1978, 25(1): 31-3).

The amount of a compound of the invention that will be effective in the treatment of a particular disorder or condition, including cancer, will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. A preferred dosage will be within the range of 0.01-1000 mg/kg of body weight, more preferably, 0.1mg/kg to 100 mg/kg and even more preferably 1 mg/kg to 10mg/kg. Effective doses may be extrapolated from dose-response curves derived from in vitro or animal model test bioassays or systems.

The following examples are presented in order to more fully illustrate certain embodiments of the invention. They should in no way, however, be construed as limiting the broad scope of the invention. One skilled in the art can readily devise many variations and modifications of the principles disclosed herein without departing from the scope of the invention.

### EXAMPLES

### EXAMPLE 1 - Cytotoxicity of Jasmonate Derivatives Towards Leukemia Cells

New jasmonate-derivatives (compounds 1-11) were tested for cytotoxicity in 3 cancer cell lines:
A) Molt-4 - Human acute lymphoblastic leukemia cell-line
B) CT26 - Marine colon carcinoma cell-line
C) MCF7 - Human breast adenocarcinoma cell-line

The new derivatives were also tested on normal lymphocytes (PBL) obtained from healthy donors and stimulated with Phytohemagglutinine (PHA) and TPA. The experimental set up as well as IC50 values obtained for the different cell lines are listed below.

### Experimental set up:

Mononuclear cells were isolated from peripheral blood of healthy donors by ficoll-hypaque density gradient centrifugation. The mononuclear cells were allowed to adhere to plastic dishes to remove macrophages. The non-adherent peripheral blood lymphocytes (PBL) were pre-incubated with 0.8 µg/mL PHA and 5 ng/mL TPA for 24 hours and then used in further experiments.

Cell densities were as follows: Molt-4 (at 2.5 X10⁴ cells in 100 µL per well), CT26 (at 5 X10³ cells in 100 µL per well), MCF7 (at 5 X10³ cells in 100 µL per well) and PBL (at 1.5 X10⁵ cells in 100 µL per well) seeded in 96-well plates. Adherent cells (CT26 and MCF7) were allowed to adhere over-night.

Jasmonate-derivatives were added at concentration ranging from 0.005-0.5 mM for 24 hours. Each experimental point was performed in triplicates. Untreated cells were used as control. The jasmonate-derivatives were prepared as a stock of 167 mM in 100% ethanol. Dilutions were performed in culture medium and ethanol so that the final concentration of ethanol in each well was 0.6%. This concentration of ethanol by itself did not affect the viability of any of the cell lines.

Optical density representing viable cells was determined using the XTT Cell Proliferation Kit assay (Biological industries, Beit-Haemek, Israel).

Percentage of Optical density is directly proportional to the number of living cells in culture. Cytotoxicity (%) was calculated in the following way: [(OD of control cells-OD of drug-treated cells)/OD of control cells]×100.

### Results

IC 50 values for the different compounds in different cell lines are listed in Table 1 below, in which compounds 1-8 and 10-11 which do not form part of the present invention.

**Table 1**

| **Compound** | **IC50 in Molt-4 (mM)** | **IC50 in CT-26 (mM)** | **IC50 in MCF-7 (mM)** | **IC50 in PBL** | **Remarks** |
|---|---|---|---|---|---|
| **1** | 0.210 ± 0.111 | 0.307 ± 0.167 | 0.220 ± 0.000 | >0.500 | |
| **2** | 0.440 ± 0.109 | >0.500 | >0.500 | >0.500 | |
| **3** | 0.340 ± 0.226 | 0.340 ± 0.226 | >0.500 | Not done | |
| **4** | 0.037 ± 0.038 | 0.135 ± 0.049 | 0.070 ± 0.000 | 0.060 ± 0.014 | |
| **5** | 0.086 ± 0.058 | 0.430 ± 0.099 | >0.500 | 0.220 ± 0.000 | |
| **6** | 0.056 ± 0.011 | 0.263 ± 0.212 | 0.236 ± 0.180 | 0.090 ± 0.014 | |
| **7** | 0.125 ± 0.026 | 0.393 ± 0.141 | 0.323 ± 0.075 | 0.425 ± 0.021 | |
| **8** | 0.050 ± 0.028 | 0.163 ± 0.042 | 0.120 ± 0.060 | 0.06 ± 0.014 | |
| **9** | 0.012 ± 0.009 | 0.029 ± 0.021 | 0.060 ± 0.014 | 0.200 ± 0.085 | |
| **10** | 0.243 ± 0.039 | >0.5 | - | >0.5 | |
| **11** | 0.079 ± 0.006 | 0.237 ± 0.029 | 0.110 ± 0.020 | 0.323 ± 0.211 | |

### EXAMPLE 2 - Selectivity of Jasmonate Derivatives: Compound 9 and Compound 11

### Experimental set up

Mononuclear cells were isolated from peripheral blood of healthy donors and treated as described above. The non-adherent PBL were pre-incubated with PHA and TPA as described above. Molt-4 and PBL cells were seeded in 96-well plates as described above.

Jasmonate derivatives, compound 9 and compound 11, compound 11 not forming part of the present invention, were added at concentration ranging from 0.005-0.5 mM for 24 hours. Each experimental point was performed in triplicates. Untreated cells were used as control. The Jasmonate-derivatives were prepared as a stock of 167 mM in 100% ethanol and dilutions in medium were prepared as described above. Optical density and percentage of Cytotoxicity were determined as described above.

As shown in Figure 1A and 1B, there is a comfortable therapeutic window which allows compounds 9 and 11 to kill leukemic cells without exerting a substantial effect on normal lymphocytes. The results demonstrate the ability of the compounds described herein to exert a selective cytotoxic effect against cancer cells, without substantially affecting normal cells.

### EXAMPLE 3- Effect of compound 9 tested in vitro in a clonogenic assay (TCA) in different patients' derived tumors

### Experimental set up

Cancer cells were taken directly from cancer patients, employing the Tumor-Colony-Assay: Solid human tumor xenografts growing subcutaneously in serial passages in thymus aplastic nude mice were removed and disaggregated to obtain isolated tumor cells. Viable cells were added to culture medium supplemented with agar and plated in 24-multiwell dishes. The test compound was applied in the cultures that were incubated at 37°C for 6-20 days and monitored for colony growth using an inverted microscope. At the time of maximum colony formation, colonies were counted and 24 hours prior to evaluation, stained with a vital dye. Drug effects were expressed in terms of the percentage of colony formation, obtained by comparison of the mean number of colonies in the treated wells with the mean colony count of untreated controls. IC₅₀ values, being the drug concentrations necessary to inhibit colony formation by 50%, were determined by plotting compound concentration versus relative colony count. Results are depicted in Table 2.

**Table 2. Effect of Compound 9 tested in vitro in a clonogenic assay (TCA) in different patients' derived tumors**

| **Tissue origin** | **Tumor model** | **Histology** | **IC50 (µM)** |
|---|---|---|---|
| Colon | CXF 1299 | Adeno carcinoma | 1.12 |
| Colon | CXF 280 | Adeno carcinoma, ud | 5.44 |
| Stomach | GXF 214 | Adeno carcinoma, ud | 1.94 |
| Lung, non small | LXFA 1012 | Adeno carcinoma, pd | 1.32 |
| Lung, non small | LXFA 1041 | Adeno carcinoma, md | 1.50 |
| Lung, non small | LXFA 629 | Adeno carcinoma, pd | 1.38 |
| Lung, non small | LXFA 749 | Adeno carcinoma, pd | 1.70 |
| Lung, non small | LXFL 430 | Large cell lung carcinoma, ud | < 1.00 |
| Lung, small cell | LXFS 573 | Small cell lung carcinoma | 1.93 |
| Breast | MAXF 1322 | Papillary adeno carcinoma, pd | 1.55 |
| Breast | MAXF 857 | Invasive ductal carcinoma | 1.55 |
| Kidney | RXF 1220 | Hypernephroma, pd | 6.32 |
| Kidney | RXF 423 | Hypernephroid adeno carcinoma | 1.69 |

| | | | |
|---|---|---|---|
| md : moderately differentiated, pd : poorly differentiated, ud : undifferentiated | | | |

Comparing these values to the results with cell lines (Table 1) it is evident that compound 9 is even more potent against patient-derived tumors than against cell lines.

### EXAMPLE 4- Ability of compound 9 to induce a decrease in the ATP levels of CT-26 colon carcinoma cells

The ability of compound 9 to induce a decrease in the ATP levels of CT-26 colon carcinoma cells was evaluated. It was previously found that the natural jasmonate methyl jasmonate can cause a decrease in ATP levels in cancer cells (Fingrut et al., 2005 Fingrut O, et al. Br. J. Pharmacol. 146:800-808, 2005). However, these experiments were performed under conditions (dosage and length of exposure) at which no cytotoxic effects were evident since dead cells would not contain ATP. The purpose of the present experiments was to evaluate whether a drop in ATP precedes death.

### Experimental set up

CT-26 cells were exposed to compound 9 for 3 hours and ATP levels were measured using the The CellTiter-Glot Luminescent Cell Viability Assay (Promega, Madison, WI, U.S.A.). As can be seen in figure 2, compound 9 induced a significant drop in cellular ATP levels. Furthermore, this effect was decreased in the presence of high glucose levels, suggesting that glycolysis can compensate for the ATP decrease induced by compound 9, again similar to our findings with methyl jasmonate (Fingrut et al, 2005). These data suggest similarities between the mechanism of action of the natural methyl jasmonate and its novel chemical derivative compound 9. It thus appears that a drop in cellular energy levels may precipitate the cytotoxic effect of compound 9.

### EXAMPLE 5- The ability of compound 9 to decrease experimental metastasis of B16 melanoma cells to the lungs In Vivo

The ability of compound 9 to decrease experimental metastasis of B16 melanoma cells to the lungs in vivo was determined.

### Experimental set up

The experiment was comprised of 3 groups of C57bl mice (12-15 weeks old, n=12), total of 36 all inoculated with B16F10 melanoma cells, 0.1ml of B16F10 in PBSx1, inoculums of 7x10⁵ cells per mouse. Group M1 - non treated, Group M2 - treated once daily, 5 days a week for 3 weeks with Compound 9 dissolved with surfactants (10mg/kg, diluted 1:5 with Saline, i.v.), and Group M3 - a positive control group treated with Paclitaxel once in 7 days (15mg/kg, i.v.). After 22 days, mice were sacrificed and lungs were weighed. Lungs with metastases weigh more than normal lungs (which weigh about 200 mg), and a decrease in lung weight in comparison to control tumor-bearing untreated mice signifies an anti-metastatic effect. As can be seen in Figure 3, compound 9 was very effective in suppressing lung metastasis and a t test yielded a P value of 0.000003 for compound 9 versus the control. The data suggest that compound 9 is a very promising new anti-cancer agent.

### EXAMPLE 6 - Synthesis

Exemplary synthetic methods for the preparation compound 9 of the invention as well as exemplary compound of formula 11 described herein, are set forth below.

### Compound 9:

To a stirred solution of 8-hydroxy quinoline (620 mg, 4.27 mmol) and triethylamine (0.7 mL, 5.12 mmol) in dry THF (20 mL), at 0°C under argon atmosphere was added dropwise a solution of Jasmonyl chloride (900 mg, 3.94 mmol) in dry THF (20 mL) and the reaction mixture was stirred for 1.5 hr at 0°C, allowed to warm up to room temperature and further stirred for 1 hr. The solvent was then evaporated and the residue diluted with CH₂Cl₂ and washed with saturated aq. NaHCO₃ (x 2). The organic layer was dried over MgSO₄ and concentrated in vacuo. The residue was purified by VLC (EtOAc/petroleum ether 1: 9) affording compound 9 (728 mg, 55%) as a yellow oil.

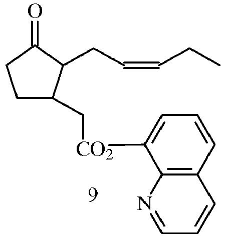

### Compound 11:

Compound 11 does not form part of the present invention. A solution of anhydrous D-glucose (159 mg, 0.883 mmol) in pyridine (10 mL) was heated to 100°C for 1 hr. The solution was allowed to cool to room temperature and a solution of compound 9 (595 mg, 1.76 mmol) in pyridine (10 mL) and NaH (5 mg of 60% NaH in oil, washed with petroleum ether before use, 0.132 mmol). The reaction mixture was heated to 60°C and stirred for 4 hours. The solvent was then evaporated and the residue diluted with n-butanol and extracted with an aqueous solution of K₃PO₄ (0.1N). The organic layer was washed with water containing few drops of AcOH and concentrated in vacuo. The residue was purified by VLC (EtOAc) affording compound 11 (45 mg, 14%) as a yellow oil.

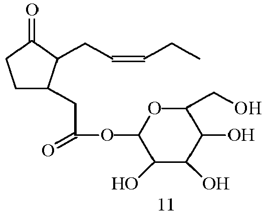

## Claims

1. A compound represented by the structure of Formula I: 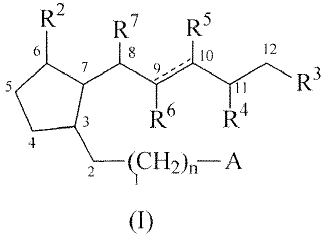 Wherein in Formula I:
A is COR¹;
R¹ is a heteroaryloxy;
R² is selected from the group consisting of hydrogen, unsubstituted or substituted C₁-C₁₂ alkyl, unsubstituted or substituted C₃-C₈ cycloalkyl, unsubstituted or substituted aryl, unsubstituted or substituted heteroaryl, OR⁸, oxo and NR^{9a}R^{9b};
R³, R⁴, R⁵, R⁶ and R⁷ are each independently selected from the group consisting of hydrogen, unsubstituted or substituted C₁-C₁₂ alkyl, unsubstituted or substituted C₁-C₁₂ haloalkyl, unsubstituted or substituted C₃-C₈ cycloalkyl, unsubstituted or substituted aryl, unsubstituted or substituted heteroaryl, OR⁸ and NR^{9a}R^{9b},
or and R⁶ together with the carbons to which they are attached form a C₃-C₈ cycloalkyl or a C₃-C₈ cycloalkyl substituted by halo;
or one of R⁵ and R⁶ represents an oxygen atom which is bonded to C₆, thereby forming an oxygen-containing 6 or 5 membered heterocyclic ring, respectively;
wherein the bond between C₉ and C₁₀ can be a single or double bond;
R⁸, R^{9a} and R^{9b} are each independently selected from the group consisting of hydrogen, unsubstituted or substituted C₁-C₁₂ alkyl, unsubstituted or substituted C₃-C₈ cycloalkyl, unsubstituted or substituted aryl, unsubstituted or substituted heteroaryl, glucosyl, or R^{9a} and R^{9b} can together with the nitrogen to which they are attached form an unsubstituted or substituted heterocyclic or heteroaromatic ring optionally containing one or more additional heteroatom selected from O, N and S; and
n is selected from O. 1 and 2;
including salts, hydrates, solvates, polymorphs, optical isomers, geometrical isomers, enantiomers, diastereomers, and mixtures thereof.

2. The compound of claim 1, wherein the heteroaryloxy is unsubstituted or substituted with one or more alkyl groups.

3. The compound of claim 1, wherein R¹ is quinolinyloxy.

4. The compound of claim 1, wherein wherein R⁵ and R⁶ together with the carbons to which they are attached form a C₃-C₈ cycloalkyl substituted by halo.

5. The compound of claim 1, wherein R² is oxo.

6. The compound of claim 1, wherein the bond between C₉ and C₁₀ is a double bond, and R³, R⁴, R⁵, R⁶ and R⁷ are each hydrogen.

7. The compound of claim 1, wherein the bond between C₉ and C₁₀ is a single bond, and R³, R⁴, R⁵, R⁶ and R⁷ are each hydrogen.

8. The compound of claim 1, wherein R⁶ represents an oxygen atom which is bonded to C₆, thereby forming an oxygen-containing 5-membered heterocyclic ring.

9. The compound of claim 1, which is represented by the structure of Formula 9:

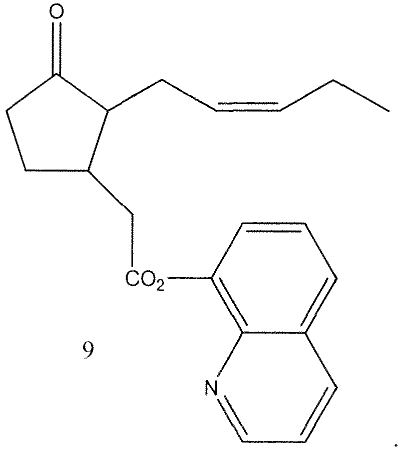

10. A pharmaceutical composition comprising a pharmaceutically acceptable carrier, and as an active ingredient a compound according to any of the claims 1 to 9.

11. The pharmaceutical composition of claim 10, wherein the composition is in a form suitable for oral administration, intravenous administration by injection, topical administration, administration by inhalation, or administration via a suppository.

12. A method for inhibiting cancer cell proliferation in vitro, comprising contacting said cancer cells with a compound according to any of the claims 1 to 9.

13. A compound according to any of the claims 1 to 9 for use in the treatment of cancer or a premalignant condition in a subject.

14. The compound according to claim 13, wherein the subject is a mammal, preferably a human.

15. The method according to claim 12, or the compound according to claim 13,
wherein the cancer is selected from the group consisting of: lymphoproliferative disorders, breast cancer, ovarian cancer, lung cancer, prostate cancer, cervical cancer, endometrial cancer, bone cancer, liver cancer, stomach cancer, colon cancer, pancreatic cancer, cancer of the thyroid, head and neck cancer, cancer of the central nervous system, cancer of the peripheral nervous system, skin cancer, kidney cancer, as well as metastases of all the above; or
wherein the cancer is selected from the group consisting of: hepatocellular carcinoma, hepatoma, hepatoblastoma, rhabdomyosarcoma, esophageal carcinoma, thyroid carcinoma, ganglioblastoma, fibrosarcoma, myxosarcoma, liposarcoma, chondrosarcoma, osteogenic sarcoma, chordoma, angiosarcoma, endotheliosarcoma, Ewing's tumor, leimyosarcoma, rhabdotheliosarcoma, invasive ductal carcinoma, papillary adenocarcinoma, melanoma, squamous cell carcinoma, basal cell carcinoma, adenocarcinoma (well differentiated, moderately differentiated, poorly differentiated or undifferentiated), renal cell carcinoma, hypernephroma, hypernephroid adenocarcinoma, bile duct carcinoma, choriocarcinoma, seminoma, embryonal carcinoma, Wilms' tumor, testicular tumor, lung carcinoma including small cell, non-small and large cell lung carcinoma, bladder carcinoma, glioma, astrocyoma, medulloblastoma, craniopharyngioma, ependymoma, pinealoma, retinoblastoma, neuroblastoma, colon carcinoma, rectal carcinoma, hematopoietic malignancies including all types of leukemia and lymphoma including: acute myelogenous leukemia, acute myelocytic leukemia, acute lymphocytic leukemia, chronic myelogenous leukemia, chronic lymphocytic leukemia, mast cell leukemia, lymphoblastic leukemia, myeloma, multiple myeloma, myeloid lymphoma, Hodgkin's lymphoma, non-Hodgkin's lymphoma.

## Patentansprüche

1. Verbindung der Formel I: 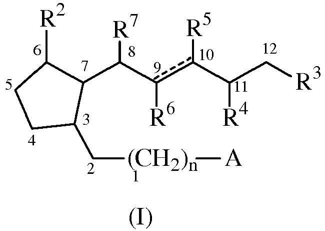 worin in Formel I:
A COR¹ ist;
R¹ ein Heteroaryloxy ist;
R² ausgewählt ist aus der Gruppe bestehend aus Wasserstoff, unsubstituiertem oder substituiertem C₁-C₁₂ Alkyl, unsubstituiertem oder substituiertem C₃-C₈ Cycloalkyl, unsubstituiertem oder substituiertem Aryl, unsubstituiertem oder substituiertem Heteroaryl, OR⁸, Oxo und NR^{9a}R^{9b};
R³, R⁴, R⁵, R⁶ und R⁷ unabhängig voneinander ausgewählt sind aus der Gruppe bestehend aus Wasserstoff, unsubstituiertem oder substituiertem C₁-C₁₂ Alkyl, unsubstituiertem oder substituiertem C₁-C₁₂ Haloalkyl, unsubstituiertem oder substituiertem C₃-C₈ Cycloalkyl, unsubstituiertem oder substituiertem Aryl, unsubstituiertem oder substituiertem Heteroaryl, OR⁸ und NR^{9a}R^{9b},
oder R⁵ und R⁶ zusammen mit den Kohlenstoffatomen, an denen sie angeordnet sind, einen C₃-C₈ Cycloalkyl oder einen C₃-C₈ Cycloalkyl substituiert mit Halogen bilden;
oder einer von R⁵ und R⁶ ein Sauerstoffatom repräsentiert, das an C₆ gebunden ist, wodurch ein heterozyklischer Ring mit 6 oder 5 Elementen gebildet wird, der Sauerstoff enthält;
worin die Bindung zwischen C₉ und C₁₀ eine Einzel- oder Doppelbindung sein kann;
R⁸, R^{9a} und R^{9b} unabhängig voneinander ausgewählt sind aus der Gruppe bestehend aus Wasserstoff, unsubstituiertem oder substituiertem C₁-C₁₂ Alkyl, unsubstituiertem oder substituiertem C₃-C₈ Cycloalkyl, unsubstituiertem oder substituiertem Aryl, unsubstituiertem oder substituiertem Heteroaryl, Glukosyl, oder R^{9a} und R^{9b} bilden zusammen mit dem Stickstoffatom, an dem sie angeordnet sind, einen unsubstituierten oder substituierten heterozyklischen oder heteroaromatischen Ring, der wahlweise ein oder mehrere Heteroatome ausgewählt unter O, N und S enthält; und
n ausgewählt ist unter 0, 1 und 2;
einschließlich Salzen, Hydraten, Solvaten, Polymorphe, optische Isomere, geometrische Isomere, Enantiomere, Diastereomere und Gemischen davon.

2. Verbindung nach Anspruch 1, worin das Heteroaryloxy mit einer oder mehreren Alkylgruppen unsubstituiert oder substituiert ist.

3. Verbindung nach Anspruch 1, worin R¹ Chinolinyloxy ist.

4. Verbindung nach Anspruch 1, worin R⁵ und R⁶ zusammen mit den Kohlenstoffatomen, an denen sie angeordnet sind, einen C₃-C₈ Cycloalkyl substituiert mit Halogen bilden.

5. Verbindung nach Anspruch 1, worin R² Oxo ist.

6. Verbindung nach Anspruch 1, worin die Bindung zwischen C₉ und C₁₀ eine Doppelbindung ist, und R³, R⁴, R⁵, R⁶ und R⁷ jeweils Wasserstoff sind.

7. Verbindung nach Anspruch 1, worin die Bindung zwischen C₉ und C₁₀ eine Einzelbindung ist, und R³, R⁴, R⁵, R⁶ und R⁷ jeweils Wasserstoff sind.

8. Verbindung nach Anspruch 1, worin R⁶ ein Sauerstoffatom repräsentiert, das an C₆ gebunden ist, wodurch ein heterozyklischer Ring mit 5 Elementen gebildet wird, der Sauerstoff enthält.

9. Verbindung nach Anspruch 1, dargestellt durch die Struktur der Formel 9:

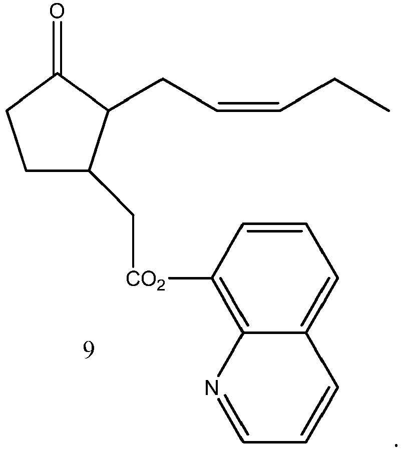

10. Pharmazeutische Zusammensetzung umfassend einen pharmazeutisch verträglichen Träger und als aktiven Bestandteil eine Verbindung nach einem der Ansprüche 1 bis 9.

11. Pharmazeutische Zusammensetzung nach Anspruch 10, worin die Zusammensetzung in einer Form vorliegt, die geeignet ist für eine orale Verabreichung, intravenöse Verabreichung mittels Injektion, topische Verabreichung, Verabreichung durch Inhalation oder eine Verabreichung über Zäpfchen.

12. Verfahren zum Inhibieren von Krebszellenproliferationen in vitro, umfassend ein Inkontaktbringen der Krebszellen mit einer Verbindung nach einem der Ansprüche 1 bis 9.

13. Verbindung nach einem der Ansprüche 1 bis 9 zur Verwendung in der Behandlung von Krebs oder eines prämalignen Zustandes eines Subjekts.

14. Verbindung nach Anspruch 13, wobei das Subjekt ein Säugetier ist, vorzugsweise ein Mensch.

15. Verfahren nach Anspruch 12 oder Verbindung nach Anspruch 13,
wobei der Krebs ausgewählt ist aus der Gruppe bestehend aus: lymphproliferativen Störungen, Brustkrebs, Eierstockkrebs, Lungenkrebs, Prostatakrebs, endometrialem Krebs, Knochenkrebs, Leberkrebs, Magenkrebs, Dickdarmkrebs, Bauchspeicheldrüsenkrebs, Schilddrüsenkrebs, Kopf- und Halskrebs, Krebs des zentralen Nervensystems, Krebs des peripheren Nervensystems, Hautkrebs, Nierenkrebs, sowie Metastasen der vorstehenden, oder
wobei der Krebs ausgewählt ist aus der Gruppe bestehend aus: Leberzellenkarzinom, Hepatom, Hepatoblastom, Rhabdomyosarkom, Ösophagealkarzinom, Schilddrüsenkarzinom, Ganglioblastom, Fibrosarkom, Myxosarkom, Liposarkom, Chondrosarkom, Osteosarkom, Chrordom, Angiosarkom, Endothelsarkom, Ewing-Sarkom, Leimyosarkom, Rhabdotelsarkom, invasives duktales Karzinom, papilläres Adenokarzinom, Melanom, Plattenepithelkarzinom, Basalzellenkarzinom, Adenokarzinom (stark abgegrenzt, leicht abgegrenzt, schwach abgegrenzt oder gar nicht abgegrenzt), Nierenzellkarzinom, Hypernephrom, hypernephroides Adenokarzinom, Gallengangkarzinom, Chorionkarzinom, Seminom, Wilm-Tumor, Hodentumor, Lungenkarzinom einschließlich Karzinome der kleinen Lungenzellen, der nicht kleinen und der großen Lungenzellen, Blasenkarzinom, Gliom, Astrozytom, Medulloblastom, Kraniopharyngeom, Ependymom, Pinealom, Retinoblastom, Neuroblastom, Kolonkarzinom, Rektalkarzinom, hämatopoetische Malignitäten einschließlich alle Arten von Leukämie und Lymphome einschließlich: akute lymphotische Leukämie, chronische myeloische Leukämie, chronische lymphotische Leukämie, Mastzellenleukämie, lymphoblastische Leukämie, multiples Myelom, myeloisches Lymphom, Hodgkin-Lymphom, nicht-Hodgkin-Lymphom.

## Revendications

1. Un composé représenté par la structure de la Formule I : 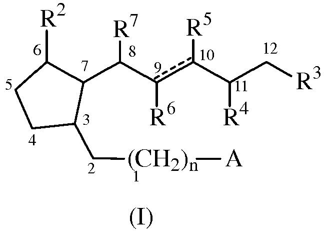 Formule I où :
A est COR¹ ;
R¹ est un hétéroaryloxy ;
R² est sélectionné du groupe consistant en hydrogène, alkyl CᵣC₁₂ non substitué ou substitué, cycloalkyl C₃-C₈ non substitué ou substitué, aryl non substitué ou substitué, hétéroaryloxy non substitué ou substitué, OR⁸, oxo et NR^{9a}R^{9b} ;
R³, R⁴, R⁵, R⁶ et R⁷ sont chacun indépendamment sélectionné du groupe consistant en hydrogène, alkyl C₁-C₁₂ non substitué ou substitué, haloalkyl C₁-C₂ non substitué ou substitué, cycloalkyl C₃-C₈ non substitué ou substitué, aryl non substitué ou substitué, hétéroaryl non substitué ou substitué, OR⁸ et NR^{9a}R^{9b},
ou R⁵ et R⁶ avec les carbones auxquels ils sont liés forment un cycloalkyl C₃-C₈ ou un cycloalkyl C₃-C₈ substitué par halo ;
ou un de R³ et R⁶ représente un atome d'oxygène qui est lié à C₆, formant ainsi un noyau hétérocyclique à 5 ou 6 chaînon contenant de l'oxygène, respectivement ;
où le lien entre C₉ et C₁₀ peut être un lien simple ou double ;
R⁸, R^{9a} et R^{9b} sont chacun indépendamment sélectionnés du groupe consistant en hydrogène, alkyl C₁-C₁₂ non substitué ou substitué, cycloalkyl C₃-C₈ non substitué ou substitué, aryl non substitué ou substitué, hétéroaryl non substitué ou substitué, glucosyl, ou R^{9a} et R^{9b} peuvent avec le nitrogène auquel ils sont liés former un noyau hétérocyclique ou hétéroaromatique non substitué ou substitué contenant optionnellement un ou plusieurs hétéroatome additionnels sélectionnés de O, N et S ; et n est sélectionné de 0, 1 et 2 ;
comprenant des sels, hydrates, solvates, polymorphes, isomères optiques, isomères géométriques, énantiomères, diastéréomères et mélanges de ceux-ci.

2. Le composé de la revendication 1, où l'hétéroaryloxy est non substitué ou substitué par un ou plusieurs groupes alkyl.

3. Le composé de la revendication 1, où R¹ est quinolinyloxy.

4. Le composé de la revendication 1, où R⁵ et R⁶ avec les carbones auxquels ils sont liés forment un cycloalkyl C₃-C₈ substitué par halo.

5. Le composé de la revendication 1, où R² est oxo.

6. Le composé de la revendication 1, où le lien entre C₉ et C₁₀ est un lien double, et R³, R⁴, R⁵, R⁶ et R⁷ sont chacun de l'hydrogène.

7. Le composé de la revendication 1, où le lien entre C₉ et C₁₀ est un lien unique, et R³, R⁴, R⁵, R⁶ et R⁷ sont chacun de l'hydrogène.

8. Le composé de la revendication 1, où R⁶ représente un atome d'oxygène qui est lié à C₆, formant ainsi un noyau hétérocyclique à 5 membres contenant de l'oxygène.

9. Le composé de la revendication 1, qui est représenté par la structure de la Formule 9 :

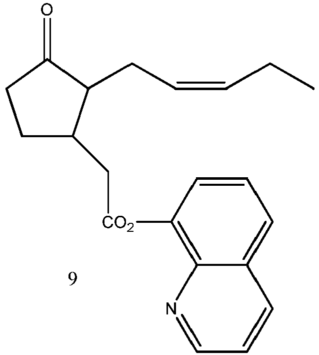

10. Une composition pharmaceutique comprenant un porteur pharmaceutiquement acceptable, et comme ingrédient actif un composé conformément à n'importe laquelle des revendications 1 à 9.

11. La composition pharmaceutique de la revendication 10, où la composition est une forme appropriée à l'administration orale, l'administration intraveineuse par injection, l'administration topique, l'administration par inhalation ou l'administration par suppositoire.

12. Une méthode destinée à inhiber la prolifération des cellules cancéreuses in vitro, comprenant la mise en contact des cellules cancéreuses en question avec un composé conformément à n'importe laquelle des revendications 1 à 9.

13. Un composé conformément à n'importe laquelle des revendications 1 à 9 à utiliser dans le traitement du cancer ou d'une condition pré-maligne chez un sujet.

14. Le composé d'après la revendication 13, où le sujet est un mammifère, de préférence un humain.

15. La méthode d'après la revendication 12, ou le composé d'après la revendication 13,
où le cancer est sélectionné du groupe consistant en :
troubles lymphoprolifératifs, cancer du sein, cancer des ovaires, cancer du poumon, cancer de la prostate, cancer de l'utérus, cancer endométrial, cancer des os, cancer du foie, cancer de l'estomac, cancer du colon, cancer du pancréas, cancer de la thyroïde, cancer de la tête et du cou, cancer du système nerveux central, cancer du système nerveux périphérique, cancer de la peau, cancer du rein ainsi que métastases de tout ce qui est mentionné ci-dessus ; ou
où le cancer est sélectionné du groupe consistant en : carcinome hépatocellulaire, hépatome, hépatoblastome, rhabdomyosarcome, carcinome de l'oesophage, carcinome de la thyroïde, ganglioblastome, fibrosarcome, myxosarcome, liposarcome, chondrosarcome, sarcome ostéogénique, chordome, angiosarcome, endothéliosarcome, tumeur d'Ewing, léimyosarcome, rhabdothéliosarcome, carcinome ductal envahisseur, adénocarcinome papillaire, mélanome, carcinome cellulaire squameux, carcinome de la cellule basale, adénocarcinome (bien différencié, modérément différencié, très peu différencié ou non différencié), carcinome cellulaire rénal, hypernéphrome, adénocarcinome hypernéphroïde, carcinome du conduit de la bile, choriocarcinome, seminome, carcinome embryonnaire, tumeur de Wilms, tumeur testiculaire, carcinome du poumon, y compris carcinome du poumon à petites, non-petites et grandes cellules, carcinome de la vessie, gliome, astrocyome, médulloblastome, craniopharyngiome, épendymome, pinéalome, rétinoblastome, neuroblastome, carcinome du colon, carcinome rectal, malignités hématopoïétiques comprenant tous les types de leucémie et lymphome, y compris : leucémie myélogéneuse aiguë, leucémie myélocytique aiguë, leucémie lymphocytique aiguë, leucémie myélogéneuse chronique, leucémie lymphocytique chronique, leucémie à mastocytes, leucémie lymphoblastique, myélome, myélome multiple, lymphome myéloïde, lymphome de Hodgkin, lymphome non hodgkinien.
